# EUROPÄISCHE PATENTSCHRIFT

(11) **EP 3 438 225 B1**
(45) Veröffentlichungstag und Bekanntmachung des Hinweises auf die Patenterteilung: **25.11.2020**
(21) Anmeldenummer: 18185804.4
(22) Anmeldetag: 26.07.2018
(51) Int. Cl.: C09K 5/06, C08F 220/58

(54) **LATENTWÄRMESPEICHERMEDIUM**
LATENT HEAT STORAGE MEDIUM
MOYEN D'ACCUMULATION DE CHALEUR LATENTE

(30) Priorität: 03.08.2017 DE 102017117599
(43) Veröffentlichungstag der Anmeldung: 06.02.2019
(73) Patentinhaber: i-select SA, 2000 Neuchâtel (CH)
(72) Erfinder: ANDERS, Olaf, 37216 Witzenhausen (DE)
(74) Vertreter: Lambacher, Michael

(56) Entgegenhaltungen:
- AT-B- 379 407
- DE-A1- 3 124 008
- DE-T2- 69 513 895
- DE-T2- 69 737 519
- US-E- R E35 586

## Beschreibung

### Gebiet der Erfindung

Die Erfindung betrifft ein Additiv zur Stabilisierung von Natriumacetat-Trihydrat, sowie ein Verfahren zur Herstellung dieses Additivs. Die Erfindung betrifft ferner ein Speichermedium für einen Latentwärmespeicher, umfassend Natriumacetat-Trihydrat und das Additiv, sowie ein Verfahren zur Stabilisierung dieses Speichermediums.

### Hintergrund der Erfindung

Latentwärmespeicher sind im Stand der Technik bekannt. Diese besitzen die Fähigkeit, die thermische Energie über einen langen Zeitraum und mit einer Vielzahl von Wiederholzyklen zu speichern. Das Grundprinzip des Latentwärmespeichers beruht auf der Ausnutzung der Enthalpie thermodynamischer Zustandsänderungen, wie z.B. dem Phasenübergang von flüssig nach fest und umgekehrt. Zum Einsatz kommt in Latentwärmespeichern häufig Natriumacetat-Trihydrat (CH₃COONa ^{∗} 3 H₂O), ein in Wasser bei 20°C gut lösliches farbloses Salzhydrat.

Natriumacetat-Trihydrat ist bei Raumtemperatur eine feste Substanz. Es wird durch Erwärmen oberhalb der Schmelztemperatur von 58°C verflüssigt. Nach der vollständigen Verflüssigung des Materials kann dieses auf Temperaturen auch weit unterhalb des Schmelzpunktes, beispielsweise auf Raumtemperatur oder unter Umständen sogar bis auf -20°C abgekühlt werden, ohne dabei zu kristallisieren. Das Natriumacetat-Trihydrat liegt dann als unterkühlte Schmelze vor, d.h. in einem thermodynamisch metastabilen Zustand bei dem eigentlich ein Phasenübergang stattfinden sollte. Dieser Effekt wird beispielsweise in handelsüblichen Hand- bzw. Taschenwärmern genutzt. In diesen befindet sich meist ein Metallplättchen, welches nach einer Zustandsänderung dann eine Kristallisation des Materials startet und die durch die Kristallisation freigewordene Energie in Form von Wärme abgibt. Aus der US RE. 35,586 ist eine wiederverwendbare Wärmepackung aus wässrigem Natriumacetat und einem Geliermittel aus Polyacrylsäure bzw. Acrylamid-Methylpropansulfonsäure bekannt.

Weiter ist aus der DE 697 37 519 T2 ein Wärmespeichersystem mit einem Wärmespeichermaterial bekannt, welches ein Salzhydrat enthält. Weiter enthält das System einen Inhibitor der Fest-Flüssig-Trennung, zudem einen Impfkristall, ein Reglerteil, einen Hauptkörper, und einen Erhitzer. Als Inhibitor der Fest-Flüssig-Trennung wird unter anderem ein Polymerisat aus Natriumacrylat und N,N'-Methylenbisacrylamid genannt.

Die DE 695 13 895 T2 beschreibt ein Verfahren zur Verhinderung der Unterkühlung einer Latentwärmespeicherzusammensetzung umfassend ein Salzhydrat und ein Hydrogel zur Stabilisierung. In diesem Verfahren kommt ein Geliermittel, hergestellt durch Polymerisation von Natriumacrylat und N,N'-Methylenbisacrylamid zum Einsatz. Als Redoxinitiator wird Kaliumperoxidsulfat und Natriumsulfit eingesetzt.

Aus der AT 379 407 ist ein Wärmeenergiespeichermaterial auf der Basis eines hydratisierten anorganischen Salzes, z.B. Natriumacetat-Trihydrat und einem Additiv aus wasserlöslichen Polymeren mit anhängender Carbonsäure- oder Sulfonsäuregruppe bekannt. Als Vernetzer wird ein mehrwertiges Metall, insbesondere Mg oder Al beschrieben.

Die DE 31 24 008 A1 beschreibt vernetzte, in Wasser quellbare Copolymerisate und ihre Verwendung als Absorptionsmittel für wässrige Körperflüssigkeiten wie Urin, wobei die Copolymerisate aus 2-Acrylamido-2-methylpropansulfonsäure mit Acryl- und/oder Methacrylsäure bestehen. Weiter wird ein bifunktionaler Vernetzer beschrieben. Als Redoxinitiator wird Kaliumpersulfat plus Natriumpyrosulfit eingesetzt.

### Beschreibung der Erfindung

Natriumacetat-Trihydrat ist ohne weitere Zusätze zu instabil in der unterkühlten Schmelze. Versuche haben gezeigt, dass eine Spontanauslösung u. a. bei Mengen von mehr als 200 kg auftritt. Die für mögliche Anwendungen, z.B. der Speicherung von Wärme für Brauchwasser und Heizung, notwendigen Mengen benötigen daher einen Zusatz, um die Stabilität zu erhöhen.

Problematisch bei unterkühlten Schmelzen auf Basis von Natriumacetat-Trihydrat ist der Umstand, dass nach einiger Zeit und einigen Wiederholungszyklen eine Separation des Wassers auftreten kann. Des Weiteren kann es zu einer zunehmenden "Anhydrat"-bildung kommen, welche durch das zunehmende Konzentrationsgefälle noch verstärkt wird. Weiter problematisch ist, dass von diesem sogenannten Anhydrat auch eine Spontanauslösung ausgehen kann, eine gezielt herbeigeführte Kristallisation zu einem bestimmten Zeitpunkt also erschwert bis unmöglich gemacht wird.

Es gilt also eine Umgebung für das verwendete Speichermedium Natriumacetat-Trihydrat zu schaffen, welche Störfaktoren möglichst gering hält. Weiter soll die Umgebung dafür sorgen, dass eine Spontanauslösung nicht oder zumindest mit einer deutlich geringeren Wahrscheinlichkeit auftritt.

Die Stabilität der unterkühlten Schmelze und die spezifische Speicherkapazität des Natriumacetat-Trihydrats setzt voraus, dass es weder zu einer Entmischung von Salz und Wasser noch zu einer Anhydratbildung kommt.

Aufgabe der vorliegenden Erfindung ist es daher, eine Zusammensetzung bereit zu stellen bei der die oben genannten Probleme nicht auftreten oder zumindest stark reduziert werden können, um die Stabilität des Natriumacetat-Trihydrats zu erhöhen.

Die Aufgabe wird durch ein Additiv zur Stabilisierung von Natriumacetat-Trihydrat gemäß Anspruch 1 gelöst. Die Aufgabe wird ferner durch ein Verfahren zur Herstellung des Additivs gemäß Anspruch 5, ein Speichermedium für einen Latentwärmespeicher gemäß Anspruch 8, umfassend Natriumacetat-Trihydrat und das

Additiv, sowie ein Verfahren zur Stabilisierung eines Speichermediums für einen Latentwärmespeicher gemäß Anspruch 9 gelöst.

Das Additiv zur Stabilisierung von Natriumacetat-Trihydrat ist durch Emulsionspolymerisation von mindestens einem hydrophilen Monomer ausgewählt aus Acrylsäure und Acrylsäurederivaten mit einer Säuregruppe und einem Redoxinitiatorsystem erhältlich.

Dabei kann der Redoxstarter in einer Menge von 0.1% bis 10% bezogen auf die Gesamtmasse an Polymer incl. Wasser eingesetzt werden.

Dabei ist das Monomer ein Acrylsäurederivat der Sulfonsäure oder der Phosphonsäure oder ein Salz der Sulfonsäure oder Phosphonsäure.

Das Additiv kann ein Monomer sein, ausgewählt aus der Gruppe, umfassend 2-Acrylamido-2-methylpropansulfonsäure, 2-Methacrylamido-2-methylpropansulfonsäure, 2-Acrylamido-2-methyl-1-propanphosphonsäure, 2-Methacrylamido-2-methyl-1-propanphosphonsäure, 2-(Methacryloyloxy)ethyl-phosphonsäure, ein Salz davon, oder eine Mischung davon.

Bevorzugt kann das Monomer 2-Acrylamido-2-methylpropansulfonsäure sein.

Dabei kann das Additiv unter Verwendung eines Vernetzers in einer Menge von bis zu 8 mol%, bezogen auf die Menge an Monomer und Vernetzer, erhalten werden.

Der Vernetzer kann aus der Gruppe, umfassend Ethylenglycoldimethylacrylate, N,N-methylenebisacrylamide, Pentaerythritoltetraacrylate, Trimethylolpropantrimethacrylate, Trimethylolpropantriacrylate, und Mischungen davon, ausgewählt sein.

Bevorzugt liegt der Mengenbereich des Vernetzers bei 0.2 bis 0.6 mol%.

Bevorzugt kann der Vernetzer Ethylenglycoldimethacrylat sein.

Bevorzugt umfasst der Vernetzer ein Gemisch aus Ethylenglycoldimethacrylat und N,N-Methylenbisacry lamid.

Dabei kann das Gemisch aus Ethylenglycoldimethacrylat und N,N-Methylenbisacrylamid in jedem Mischungsverhältnis eingesetzt werde. Bevorzugt liegt das Mischungsverhältnis bei 1:3 bis 3:1, besonders bevorzugt bei 2:3 Ethylenglycoldimethacrylat zu N,N-Methylenbisacrylamid.

Das Redoxinitiatorsystem kann als Oxidationsmittel ein Persulfat, ein Hydroperoxid oder ein Na oder K Hypochlorid umfassen, und als Reduktionsmittel Ascorbinsäure, Formaldehyd Sulfoxylat, Tetramethylendiamin, Natriumhydroxymethylsulfinat, Kaliumpyrosulfit, Natriumhydrogensulfit, Natriumpyrosulfit, oder Mischungen davon, umfassen.

Als Redoxinitiatorsystem werden Natriumpersulfat, Kaliumpyrosulfit und Eisensulfat verwendet.

Das Additiv kann als Hydrogel vorliegen.

Erfindungsgemäß umfasst das Verfahren zur Herstellung des Additivs folgende Schritte:
- Vorlegen des Monomers in der wässrigen Phase,
- Bereitstellen der Komponenten des Redoxinitiatorsystems in wässrigen Lösungen,
- Zugeben der Komponenten des Redoxinitiatorsystems in den wässrigen Lösungen in die wässrige Phase aus Monomer, und dadurch
- Starten der Emulsionspolymerisation.

Dabei wird nach dem Vorlegen des Monomers in der wässrigen Phase ein Vernetzer zugegeben.

Dabei kann als Monomer 2-Acrylamido-2-methylpropansulfonsäure eingesetzt werden, und als Vernetzer Ethylenglycoldimethacrylat oder Ethylenglycoldimethacrylat und N,N-Methylenbisacrylamid eingesetzt werden. Als Redoxinitiatorsystem werden Natriumpersulfat, Kaliumpyrosulfit und Eisensulfat eingesetzt.

Das Additiv kann durch Emulsionspolymerisation von 2-Acrylamido-2-methylpropansulfonsäure mit Ethylenglycoldimethacrylat als Vernetzer, und Natriumpersulfat, Kaliumpyrosulfit und Eisensulfat als Redoxinitiatorsystem erhalten werden.

Weiter kann das Additiv durch Emulsionspolymerisation von 2-Acrylamido-2-methylpropansulfonsäure mit einem Gemisch aus Ethylenglycoldimethacrylat und N,N-methylenebisacrylamide als Vernetzer, und Natriumpersulfat, Kaliumpyrosulfit und Eisensulfat als Redoxinitiatorsystem erhalten werden.

Die Polymerisation des Verfahrens kann bei Raumtemperatur gestartet werden. Mit Raumtemperatur ist eine Temperatur von 22°C gemeint.

Erfindungsgemäß enthält ein Speichermedium für einen Latentwärmespeicher Natriumacetat-Trihydrat und das Additiv.

Erfindungsgemäß umfasst das Verfahren zur Stabilisierung eines Speichermediums für einen Latentwärmespeicher, wobei das Speichermedium Natriumacetat-Trihydrat und das Additiv umfasst, folgende Schritte:
- Erwärmen von festem Natriumacetat-Trihydrat und dadurch Überführen in die flüssige Phase,
- anschließend Zugeben des Additivs,
- Vermischen der Komponenten miteinander, und
- Abkühlen des erhaltenen Produkts auf Raumtemperatur.
Dabei kann das Produkt kontrolliert abkühlt werden oder man lässt das Produkt unkontrolliert abkühlen.

In dem Verfahren kann man das feste Natriumacetat-Trihydrat zum Überführen in die flüssige Phase auf eine Temperatur von über 58°C, bevorzugt auf über 65°C, noch bevorzugter auf über 78°C, besonders bevorzugt auf über 83°C erwärmen.

Normalerweise ist der vollständige Aufschmelzvorgang erst bei ca. 77°C beendet. Bei dieser Temperatur liegen H₂O und Natriumacetat in getrennter Form vor. Mit einem erfindungsgemäßen Additiv kann eine Stabilität schon bei niedrigeren Temperaturen erreicht werden.

In dem Verfahren kann das Additiv, berechnet als Trockenmasse, in einer Menge von bis zu 5 Gew.%, bevorzugt in einer Menge von 0,1 bis 2 Gew.%, besonders bevorzugt in einer Menge von 0,5 bis 1 Gew.% des Gesamtgewichts des Natriumacetat-Trihydrats zugegeben werden.

Dabei senkt jede Zugabe von H₂O bzw. Additiv die speicherbare Energiedichte des Mediums, wobei sich das Additiv positiv auf die nutzbare Temperatur auswirkt. Zusätzlich verringert zuviel Zusatzwasser auch die nutzbare Temperatur des Systems. Bevorzugt ist es deshalb mit möglichst wenig Additiv und Zusatzwasser auszukommen. Als Obergrenze kann max. 5% Additiv und 8% Zusatzwasser angesehen werden.

Das Additiv, berechnet als Trockenmasse, kann in einer Menge von 0,75 Gew.% oder 1 Gew.% zugegeben werden.

In dem Verfahren kann das Additiv als Hydrogel vorliegen. Somit wird dem Natriumacetat-Trihydrat mit dem Hydrogel auch Wasser zugegeben, welches in diesem Hydrogel inhärent enthalten ist. Ferner kann dem Natriumacetat-Trihydrat neben dem im Hydrogel inhärent enthaltenem Wasser, zusätzliches Wasser zugegeben werden.

In dem Verfahren kann man das zusätzliche Wasser direkt zum Natriumacetat-Trihydrat zugeben oder das zusätzliche Wasser wird dem Hydrogel zugeben und dann über das Hydrogel in das Natriumacetat-Trihydrat eintragen.

In dem Verfahren kann die dem Natriumacetat-Trihydrat zugegeben gesamte Menge an zusätzlichem Wasser bis zu 8 Gew.%, bevorzugt bis zu 6 Gew.%, besonders bevorzugt bis zu 5 Gew.% bezogen auf das Gewicht des Natriumacetat-Trihydrats betragen.

Experimente haben gezeigt, dass die Stabilität des Speichermediums besonders erhöht wird wenn das Additiv, berechnet als Trockenmasse, in einer Menge von 0,75 Gew.% zugegeben wird, und die gesamte Menge an zusätzlichem Wasser, welches dem Natriumacetat-Trihydrat zugegeben wird, 5 Gew.%, bezogen auf das Gewicht des Natriumacetat-Trihydrats, beträgt.

Weitere Experimente haben auch gezeigt, dass die Stabilität des Speichermediums, besonders erhöht wird wenn das Additiv, berechnet als Trockenmasse, in einer Menge von 1 Gew.% zugegeben wird, und die gesamte Menge an zusätzlichem Wasser, welches dem Natriumacetat-Trihydrat zugegeben wird, 6 Gew.%, bezogen auf das Gewicht des Natriumacetat-Trihydrats, beträgt.

Erfindungsgemäß wird das Additiv als Stabilisator für Natriumacetat-Trihydrat in einem Latentwärmespeicher verwendet.

### Ausführungsbeispiele der Erfindung

### Synthese des Additivs:

### Beispiel 1:

In diesem Beispiel werden 100 g 2-Acrylamido-2-methylpropansulfonsäure in 120 ml Wasser vorgelegt. Anschließend werden 0,18 ml Ethylenglycoldimethacrylat zugesetzt. Nach vollständiger Lösung werden 180 ml Wasser zugegeben und 0,44 g Natriumpersulfat und 0,44 g Kaliumpyrosulfit in je 20 ml Wasser gelöst. Die Natriumpersulfat-Lösung und die Kaliumpyrosulfit-Lösung werden der Lösung aus Monomer und Vernetzer zugegeben und die Polymerisation gestartet. Nach Ablauf der Reaktion wird mit 60 ml Wasser aufgefüllt.

### Beispiel 2:

In diesem Beispiel werden 100 g 2-Acrylamido-2-methylpropansulfonsäure in 120 ml Wasser vorgelegt und darin 0,23 g FeSO4 gelöst. Dann werden 0,18 ml Ethylenglycoldimethacrylat zugesetzt und nach vollständiger Lösung 180 ml Wasser zugegeben. 0,44 g Natriumpersulfat und 0,44 g Kaliumpyrosulfit werden in je 20 ml Wasser gelöst. Anschließend werden die Natriumpersulfat-Lösung und die Kaliumpyrosulfit-Lösung der Lösung aus Monomer, Vernetzer und Eisensulfat zugegeben und die Polymerisation startet. Nach Ablauf der Reaktion wird mit 60 ml Wasser aufgefüllt.

### Verwendung des Additivs:

### Beispiel 3

### Natriumacetat-Trihydrat mit 0,75% Additiv (berechnet als Trockenmasse) und 5% Extrawasser

Das gemäß Beispiel 2 hergestellte Additiv liegt als Hydrogel vor. 7,5 g dieses Hydrogels werden mit 4 ml Wasser aufgefüllt. Die daraus resultierende Mischung enthält 1,5 g Polymer, berechnet als Trockenmasse, und 10 g Wasser.

200 g festes Natriumacetat-Trihydrat werden erwärmt bis das Material vollständig verflüssigt ist. Dann wird das Additiv zugegeben und mit dem Natriumacetat-Trihydrat homogen vermischt. Anschließend stellt man das Gemisch in den Kühlschrank, kühlt es auf 4°C ab und lässt es 3 Tage im Kühlschrank stehen. Danach wird die Probe weitere 3 Tage bei Raumtemperatur gelagert. Das erhaltene Produkt ist eine klare Lösung.
Man löst die Kristallisation aus, so dass das Natriumacetat-Trihydrat auskristallisiert und die latente Wärme freigesetzt wird. Die freigesetzte Wärme wird abgeführt und die Probe dadurch wieder auf Raumtemperatur abgekühlt. Anschließend wird das feste Natriumacetat-Trihydrat wieder erwärmt und dadurch verflüssigt, und der gesamte Zyklus insgesamt 6 mal wiederholt.

### Beispiel 4

### Natriumacetat-Trihydrat mit 1% Additiv (berechnet als Trockenmasse) und 6% Extrawasser

Das gemäß Beispiel 2 hergestellte Additiv liegt als Hydrogel vor. 10 g dieses Hydrogels werden mit 4 ml Wasser aufgefüllt. Die daraus resultierende Mischung enthält 2 g Polymer, berechnet als Trockenmasse, und 12 g Wasser.

200 g festes Natriumacetat-Trihydrat werden erwärmt bis das Material vollständig verflüssigt ist. Dann wird das Additiv zugegeben und mit dem Natriumacetat-Trihydrat homogen vermischt. Anschließend stellt man das Gemisch in den Kühlschrank, kühlt es auf 4°C ab und lässt es 3 Tage im Kühlschrank stehen. Danach wird die Probe weitere 3 Tage bei Raumtemperatur gelagert. Das erhaltene Produkt ist eine klare Lösung.
Man löst die Kristallisation aus, so dass das Natriumacetat-Trihydrat auskristallisiert und die latente Wärme freigesetzt wird. Die freigesetzte Wärme wird abgeführt und die Probe dadurch wieder auf Raumtemperatur abgekühlt. Anschließend wird das feste Natriumacetat-Trihydrat wieder erwärmt und dadurch verflüssigt, und der gesamte Zyklus insgesamt 6-mal wiederholt.

## Patentansprüche

1. Additiv zur Stabilisierung von Natriumacetat-Trihydrat, erhältlich durch Emulsionspolymerisation von mindestens einem hydrophilen Monomer, wobei das Monomer ein Acrylsäurederivat der Sulfonsäure oder der Phosphonsäure oder einem Salz der Sulfonsäure oder Phosphonsäure umfasst, und einem Redoxinitiatorsystem, wobei Natriumpersulfat, Kaliumpyrosulfit und Eisensulfat als Redoxinitiatorsystem verwendet werden, und wobei das Additiv unter Verwendung eines Vernetzers erhalten wurde.

2. Additiv gemäß Anspruch 1, wobei das Monomer ausgewählt ist aus der Gruppe, umfassend 2-Acrylamido-2-methylpropansulfonsäure, 2-Methacrylamido-2-methylpropansulfonsäure, 2-Acrylamido-2-methyl-1-propanphosphonsäure, 2-Methacrylamido-2-methyl-1-propanphosphonsäure, 2-(Methacryloyloxy)ethylphosphonsäure, ein Salz davon, oder eine Mischung davon, besonders bevorzugt Acrylamido-2-methylpropansulfonsäure.

3. Additiv gemäß Anspruch 1, wobei der Vernetzer ausgewählt ist aus der Gruppe, umfassend Ethylenglycoldimethylacrylate, N,N-methylenebisacrylamide, Pentaerythritoltetraacrylate, Trimethylolpropantrimethacrylate, Trimethylolpropantriacrylate, und Mischungen davon.

4. Additiv gemäß einem der vorhergehenden Ansprüche, wobei das Additiv als Hydrogel vorliegt.

5. Verfahren zur Herstellung des Additivs gemäß einem der Ansprüche 1 bis 4, wobei das Verfahren folgende Schritte umfasst:
- Vorlegen des Monomers in der wässrigen Phase, wobei das Monomer ein Acrylsäurederivat der Sulfonsäure oder der Phosphonsäure oder einem Salz der Sulfonsäure oder Phosphonsäure umfasst,
- Bereitstellen der Komponenten des Redoxinitiatorsystems in wässrigen Lösungen, wobei Natriumpersulfat, Kaliumpyrosulfit und Eisensulfat als Redoxinitiatorsystem verwendet werden,
- Zugeben der Komponenten des Redoxinitiatorsystems in den wässrigen Lösungen in die wässrige Phase aus Monomer, und
- Starten der Emulsionspolymerisation,
- wobei nach dem Vorlegen des Monomers in der wässrigen Phase ein Vernetzer in die wässrige Phase zugegeben wird.

6. Verfahren gemäß Anspruch 5, wobei als Monomer 2-Acrylamido-2-methylpropansulfonsäure eingesetzt wird, als Vernetzer Ethylenglycoldimethacrylat oder Ethylenglycoldimethacrylat und N,N-Methylenbisacrylamid eingesetzt wird.

7. Verfahren gemäß einem der Ansprüche 5 oder 6, wobei die Polymerisation bei Raumtemperatur gestartet wird.

8. Speichermedium für einen Latentwärmespeicher, wobei das Speichermedium Natriumacetat-Trihydrat und das Additiv gemäß einem der Ansprüche 1 bis 4 umfasst.

9. Verfahren zur Stabilisierung eines Speichermediums für einen Latentwärmespeicher, wobei das Speichermedium Natriumacetat-Trihydrat und das Additiv gemäß einem der Ansprüche 1 bis 4 umfasst, und das Verfahren folgende Schritte umfasst:
- Erwärmen von festem Natriumacetat-Trihydrat und dadurch Überführen in die flüssige Phase,
- anschließend Zugeben des Additivs gemäß einem der Ansprüche 1 bis 4,
- Vermischen der Komponenten miteinander, und
- Abkühlen lassen des erhaltenen Produkts auf Raumtemperatur.

10. Verfahren gemäß Anspruch 9, wobei das feste Natriumacetat-Trihydrat zum Überführen in die flüssige Phase auf eine Temperatur von über 58°C, bevorzugt auf über 65°C, noch bevorzugter auf über 78°C, besonders bevorzugt auf über 83°C erwärmt wird.

11. Verfahren gemäß einem der Ansprüche 9 oder 10, wobei das Additiv, berechnet als Trockenmasse, in einer Menge von bis zu 5 Gew.%, bevorzugt in einer Menge von 0,1 bis 2 Gew.%, besonders bevorzugt in einer Menge von 0,5 bis 1 Gew.% des Gesamtgewichts des Natriumacetat-Trihydrats zugegeben wird.

12. Verfahren gemäß einem der Ansprüche 9 bis 11, wobei das Additiv als Hydrogel vorliegt, und wobei dem Natriumacetat-Trihydrat neben dem im Hydrogel inhärent enthaltenem Wasser, ferner zusätzliches Wasser zugegeben wird.

13. Verfahren gemäß Anspruch 12, wobei das zusätzliche Wasser direkt zum Natriumacetat-Trihydrat zugegeben wird oder das zusätzliche Wasser dem Hydrogel zugegeben wird, und dann über das Hydrogel in das Natriumacetat-Trihydrat eingetragen wird.

14. Verfahren gemäß einem der Ansprüche 12 oder 13, wobei die dem Natriumacetat-Trihydrat zugegebene gesamte Menge an zusätzlichem Wasser bis zu 8 Gew.%, bevorzugt bis zu 6 Gew.%, besonders bevorzugt bis zu 5 Gew.% bezogen auf das Gewicht des Natriumacetat-Trihydrats beträgt.

15. Verwendung des Additivs gemäß einem der Ansprüche 1 bis 4 als Stabilisator für Natriumacetat-Trihydrat in einem Latentwärmespeicher.

## Claims

1. An additive for stabilization of sodium acetate trihydrate, which can be obtained by means of emulsion polymerization of at least one hydrophilic monomer, wherein the monomer comprises an acrylic acid derivative of sulfonic acid or of phosphonic acid or a salt of sulfonic acid or phosphonic acid, and a redox initiator system, wherein sodium persulfate, potassium pyrosulfite, and iron sulfate are used as a redox initiator system, and wherein the additive was obtained using a cross-linking agent.

2. The additive according to claim 1, wherein the monomer is selected from the group comprising 2-acrylamido-2-methyl propane sulfonic acid, 2-methacrylamido-2-methyl propane sulfonic acid, 2-acrylamido-2-methyl-1-propane phosphonic acid, 2-methacrylamido-2-methyl-1-propane phosphonic acid, 2-(methacryloyloxy)ethyl-phosphonic acid, a salt thereof, or a mixture thereof, more preferably acrylamido-2-methyl propane sulfonic acid.

3. The additive according to claim 1, wherein the cross-linking agent is selected from the group comprising ethylene glycol dimethyl acrylate, N,N-methylene bisacrylamide, pentaerythritol tetra-acrylate, trimethylolpropane trimethacrylate, trimethylolpropane triacrylate, and mixtures thereof.

4. The additive according to one of the preceding claims, wherein the additive is present as a hydrogel.

5. A method for producing the additive according to one of claims 1 to 4,
wherein the method comprises the following steps:
- presenting the monomer in the aqueous phase, wherein the monomer comprises an acrylic acid derivative of sulfonic acid or of phosphonic acid or a salt of sulfonic acid or phosphonic acid
- providing the components of the redox initiator system in aqueous solutions, wherein sodium persulfate, potassium pyrosulfite, and iron sulfate are used as a redox initiator system,
- adding the components of the redox initiator system in the aqueous solutions into the aqueous phase composed of the monomer, and
- starting the emulsion polymerization,
- wherein a cross-linking agent is added to the aqueous phase after presenting the monomer in the aqueous phase.

6. The method according to claim 5, wherein 2-acrylamido-2-methyl propane sulfonic acid is used as a monomer, ethylene glycol dimethacrylate or ethylene glycol dimethacrylate and N,N-methylene bisacrylamide is used as a cross-linking agent.

7. The method according to one of claims 5 or 6, wherein the polymerization is started at room temperature.

8. A storage medium for a latent heat storage unit, wherein the storage medium comprises sodium acetate trihydrate and the additive according to one of claims 1 to 4.

9. A method for stabilization of a storage medium for a latent heat storage unit, wherein the storage medium comprises sodium acetate trihydrate and the additive according to one of claims 1 to 4, and the method comprises the following steps:
- heating of solid sodium acetate trihydrate and thereby conversion to the liquid phase,
- subsequently adding the additive according to one of claims 1 to 4,
- mixing the components with one another, and
- allowing the product obtained to cool off to room temperature.

10. The method according to claim 9, wherein the solid sodium acetate trihydrate is heated to a temperature of above 58 °C, preferably to above 65 °C, even more preferably to above 78 °C, particularly preferably to above 83 °C for conversion to the liquid phase.

11. The method according to one of claims 9 or 10, wherein the additive, calculated as dry mass, is added in an amount of up to 5 wt.-%, preferably in an amount of 0.1 to 2 wt.-%, particularly preferably in an amount of 0.5 to 1 wt.-% of the total weight of the sodium acetate trihydrate.

12. The method according to one of claims 9 to 11, wherein the additive is present as a hydrogel, and wherein additional water is added to the sodium acetate trihydrate aside from the water inherently contained in the hydrogel.

13. The method according to claim 12, wherein the additional water is added directly to the sodium acetate trihydrate, or the additional water is added to the hydrogel and then introduced into the sodium acetate trihydrate by way of the hydrogel.

14. The method according to one of claims 12 or 13, wherein the total amount of additional water added to the sodium acetate trihydrate amounts to up to 8 wt.-%, preferably up to 6 wt.-%, particularly preferably up to 5 wt.-% with reference to the weight of the sodium acetate trihydrate.

15. Use of the additive according to one of claims 1 to 4 as a stabilizer for sodium acetate trihydrate in a latent heat storage unit.

## Revendications

1. Additif destiné à stabiliser le trihydrate d'acétate de sodium, pouvant être obtenu en soumettant au moins un monomère hydrophile, ledit monomère comprenant un dérivé d'acide acrylique de l'acide sulfonique ou de l'acide phosphoreux ou d'un sel d'acide sulfonique ou d'acide phosphoreux, et un système d'initiation d'oxydoréduction à une polymérisation en émulsion, ledit système d'initiation d'oxydoréduction mis en œuvre étant constitué de persulfate de sodium, de pyrosulfite de potassium et de sulfate de fer, et ledit additif ayant été obtenu en utilisant un agent de réticulation.

2. Additif selon la revendication 1, ledit monomère étant choisi dans le groupe comprenant l'acide 2-acrylamido-2-méthylpropanesulfonique, l'acide 2-méthacrylamido-2-méthylpropanesulfonique, l'acide 2-acrylamido-2-méthyl-1 - propanephosphoreux, l'acide 2-méthacrylamido-2-méthyl-1-propanephosphoreux, l'acide 2-(méthacryloyloxy)éthylphosphoreux, l'un de leurs sels, ou l'un de leurs mélanges, s'agissant avec une préférence particulière d'acide acrylamido-2-méthylpropanesulfonique.

3. Additif selon la revendication 1, ledit agent de réticulation étant choisi dans le groupe comprenant l'éthylèneglycoldiméthylacrylate, le N,N-méthylène-bis-acrylamide, le pentaérythritoltétraacrylate, le triméthylolpropanetriméthacrylate, le triméthylolpropanetriacrylate, et leurs mélanges.

4. Additif selon l'une des revendications précédentes, ledit additif se présentant sous forme d'un hydrogel.

5. Procédé de fabrication de l'additif selon les revendications 1 à 4, ledit procédé comprenant les étapes suivantes :
- mise à disposition du monomère dans la phase aqueuse, ledit monomère comprenant un dérivé d'acide acrylique de l'acide sulfonique ou de l'acide phosphoreux ou d'un sel d'acide sulfonique ou d'acide phosphoreux,
- mise à disposition des constituants du système d'initiation d'oxydoréduction dans des solutions aqueuses, ledit système d'initiation d'oxydoréduction mis en œuvre étant constitué de persulfate de sodium, de pyrosulfite de potassium et de sulfate de fer,
- ajout des constituants dudit système d'initiation d'oxydoréduction, dans lesdites solutions aqueuses, à la phase aqueuse de monomère, et
- démarrage de la polymérisation en émulsion,
- un agent de réticulation étant ajouté à la phase aqueuse suite à la mise à disposition dudit monomère dans cette phase aqueuse.

6. Procédé selon la revendication 5, le monomère mis en œuvre étant de l'acide 2-acrylamido-2-méthylpropanesulfonique, l'agent de réticulation mis en œuvre étant de l'éthylèneglycoldiméthacrylate ou bien de l'éthylèneglycoldiméthacrylate et du N,N-méthylène-bis-acrylamide.

7. Procédé selon l'une des revendications 5 ou 6, ladite polymérisation étant démarrée à température ambiante.

8. Milieu de stockage destiné à un réservoir de chaleur latente, ledit milieu de stockage comprenant du trihydrate d'acétate de sodium et l'additif selon l'une des revendications 1 à 4.

9. Procédé de stabilisation d'un milieu de stockage destiné à un réservoir de chaleur latente, ledit milieu de stockage comprenant du trihydrate d'acétate de sodium et l'additif selon l'une des revendications 1 à 4, et le procédé comprenant les étapes suivantes :
- chauffer du trihydrate d'acétate de sodium solide pour ainsi provoquer sa transition vers la phase liquide,
- ensuite ajouter l'additif selon l'une des revendications 1 à 4,
- mélanger les constituants les uns avec les autres, et
- laisser refroidir le produit obtenu jusqu'à ce qu'il atteigne la température ambiante.

10. Procédé selon la revendication 9, le trihydrate d'acétate de sodium solide étant chauffé, pour ainsi provoquer sa transition vers la phase liquide, à une température supérieure à 58 °C, de préférence supérieure à 65 °C, de manière encore préférée supérieure à 78 °C, avec une préférence particulière supérieure à 83 °C.

11. Procédé selon l'une des revendications 9 ou 10, ledit additif étant ajouté dans une quantité, calculée à partir à sa masse sèche, pouvant aller jusqu'à 5 % en poids, de préférence dans une quantité comprise entre 0,1 et 2 % en poids, avec une préférence particulière dans une quantité comprise entre 0,5 et 1 % en poids, par rapport au poids total du trihydrate d'acétate de sodium.

12. Procédé selon l'une des revendications 9 à 11, ledit additif se présentant sous forme d'un hydrogel, et de l'eau supplémentaire étant ajoutée, outre l'eau contenue de manière inhérente dans ledit hydrogel, audit trihydrate d'acétate de sodium.

13. Procédé selon la revendication 12, ladite eau supplémentaire étant ajoutée directement audit trihydrate d'acétate de sodium, ou ladite eau supplémentaire étant ajoutée audit hydrogel pour ensuite être intégrée au trihydrate d'acétate de sodium par l'intermédiaire de cet hydrogel.

14. Procédé selon l'une des revendications 12 ou 13, la quantité totale d'eau supplémentaire qui est ajoutée audit trihydrate d'acétate de sodium pouvant atteindre jusqu'à 8 % en poids, de préférence jusqu'à 6 % en poids, avec une préférence particulière jusqu'à 5 % en poids, par rapport au poids du trihydrate d'acétate de sodium.

15. Utilisation de l'additif selon l'une des revendications 1 à 4 en tant que stabilisateur de trihydrate d'acétate de sodium dans un réservoir de chaleur latente.
